# EUROPEAN PATENT APPLICATION

(11) **EP 2 932 990 A1**
(43) Date of publication of application: **21.10.2015**
(21) Application number: 13861967.1
(22) Date of filing: 11.12.2013
(51) Int. Cl.: A61M 1/02, A61K 35/14, A61P 17/02, A61P 17/16, A61P 19/08

(54) **DEVICE FOR SEPARATING/HOUSING BLOOD COMPONENTS AND METHOD FOR PREPARING PLATELET-RICH PLASMA**

(30) Priority: 13.12.2012 JP 2012272750; 26.03.2013 JP 2013064588
(71) Applicant: JMS Co., Ltd., Hiroshima-shi, Hiroshima 730-8652 (JP)
(72) Inventor: OKAMOTO, Yasunori, Hiroshima-shi Hiroshima 730-8652 (JP); TANAKA, Seishin, Hiroshima-shi Hiroshima 730-8652 (JP); OGATA, Kasumi, Hiroshima-shi Hiroshima 730-8652 (JP)
(74) Representative: Israelsson, Stefan
(86) International application number: PCT/JP2013/083196
(87) International publication number: WO 2014/092115

(57) **Abstract**

Provided are: a device that is for separating/housing blood components and that can more easily and aseptically separate/house platelet-rich plasma; and a method for preparing platelet-rich plasma. The device (1) for separating/housing blood components is provided with: a blood storage section (2); a component housing section (3) for housing a component that is part of blood; and a linking tube (4) that joins the blood storage section (2) and the component housing section (3). The blood storage section (2) has: a tube-shaped blood storage vessel (21) that is flexible; a blood lead-in pathway (27) that leads blood into the blood storage vessel (21); and a component lead-out pathway (28) that leads out a component that is part of blood from the blood storage vessel (21). The component housing section (3) has: a component lead-in pathway (37) that leads in a component that is part of blood led out from the blood storage vessel (21); and a metal needle (371) that extends from the component lead-in pathway (37) towards the interior space of the component housing section (3) and can return the component that is part of blood housed in the component housing section (3) towards the blood storage vessel (21).

## Description

### TECHNICAL FIELD

The present invention relates to an apparatus for separating and storing blood components and a method for preparing platelet-rich plasma.

### BACKGROUND ART

Currently, in the field of regenerative medicine, studies in which stem cells collected from a subject are caused to proliferate or differentiate ex vivo, and are thereafter transplanted into a subject, thereby promoting regeneration of tissue of the subject, have been carried out. Stem cells are multipotent and can differentiate into a variety of tissues and organs, and they have been attracting attention as cells which are the key to regenerative medicine.

It has been known that in ex vivo cultural proliferation of stem cells, the addition of a serum to the medium is effective. However, when human therapies are targeted, the use of a serum derived from an animal other than humans should be avoided in light of possible safety problems. Therefore, the use of a serum prepared from blood collected from a human, in particular, collected from the same subject is desired. Furthermore, in comparison to a blood test, cultivation of stem cells in the field of regenerative medicine requires a relatively large amount of serum. In addition, in order to prepare serum assuming application to a human, it is required to separate and store the serum aseptically in a closed system.

As a response to the abovementioned various requirements, the present applicants have already presented an apparatus for separating and storing blood components that includes: a blood storage part for storing blood, and a component storage part linked aseptically and in an air-tight manner to the blood storage part, in which the blood storage part has a blood storage container of cylindrical shape having flexibility, and can introduce serum prepared from the blood into the component storage part by causing the blood storage part to deform so if being crushed (refer to Patent Document 1).

However, the platelet-rich plasma (PRP) is plasma containing high density of platelets, and due to containing growth factor in high concentration, it is being used not only in cell cultures, but in various medical fields such as wound healing, bone regeneration, and facelifts in the cosmetic field. In this way, it has been demanded that platelet-rich plasma be separated and stored aseptically in a closed system similarly to serum, due to being used also in medical fields.

Patent Document 1: Japanese Unexamined Patent Application, Publication No. 2009-50696

### DISCLOSURE OF THE INVENTION

### Problems to be Solved by the Invention

However, as a container for preparing platelet-rich plasma, although various types of containers exist, a container for preparing that satisfies requirements such as being general-purpose not requiring dedicated equipment, and aseptic separation and housing still does not exist. In addition, the apparatus for separating and storing blood components described in Patent Document 1 is optimized for the preparation of serum that is not accompanying complicated processes, and it is difficult to apply to the preparation of platelet-rich plasma, which accompanies complex processes compared to the preparation of serum.

The present invention has an object of providing an apparatus for separating and storing blood components and a method for preparing platelet-rich plasma that can separate and store platelet-rich plasma more easily and aseptically.

### Means for Solving the Problems

The present invention relates to an apparatus for separating and storing blood components, the apparatus comprising: a blood storage part for storing blood; a component storage part for storing a part of the components of the blood stored in the blood storage part; and a linking tube for aseptically linking the blood storage part and the component storage part, in which the blood storage part has a blood storage container in the form of a flexible cylinder, a blood inlet channel for introducing the blood into the blood storage container, and a component outlet channel for extracting a part of the components of the blood from the blood storage container; the component storage part has a component inlet channel for introducing a part of the components of the blood that have been extracted from the blood storage container, and a return pipe that extends from the component inlet channel towards an interior space of the component storage part, capable of sending back a part of the components of the blood stored in the component storage part to the blood storage container; and the linking tube links the component outlet channel and the component inlet channel.

It is preferable to further comprise a closing-off device that is provided to the linking tube and is for blocking movement of the part of the components of the blood within the linking tube.

It is preferable for the linking tube to include: a blood storage part-side tube having one end side connected to the blood storage part; a component storage part-side tube having one end side connected to the component storage part; and a connector that detachably links the blood storage part-side tube and the component storage part-side tube.

It is preferable to further comprise: a blood inlet tube that is connected to the blood inlet channel; and an anticoagulant introduction part that is provided to the blood inlet tube, and leads in an anticoagulant to the blood storage container.

It is preferable for the apparatus for separating and storing blood components to further comprise: a blood inlet tube having a leading end side that is connected to the blood inlet channel; and a blood inlet port that is provided to a base end side of the blood inlet tube.

It is preferable for the return pipe to be configured from a hard member.

It is preferable for an opening to be formed in one end side of the blood storage container; the blood storage part to further include a first cap that fits the opening of the blood storage container and hermetically seals the blood storage container; and the blood inlet channel and the component outlet channel to be provided to the first cap.

It is preferable for the blood storage part to further include a first storage container having a cylindrical shape and an opening formed at one end, and containing the blood storage container; the opening of the first storage container to be hermetically sealed by being fitted to the first cap; and a pressure regulation space to be formed in a space independent of an internal space of the blood storage container, between an outer side of the blood storage container and an inner side of the first storage container.

It is preferable for the blood storage part to comprise an injection hole by which fluid can be injected into the pressure regulation space.

It is preferable to further comprise an injection means, linked to the injection hole, for injecting fluid into the pressure regulation space, in which the blood storage container deforms due to the fluid that is injected.

It is preferable for the injection hole to be provided in the first cap.

It is preferable for the component storage part to comprise: a component storage container of cylindrical shape having an opening at one end, a second storage container of cylindrical shape having an opening at one end, and containing the component storage container, and a second cap connected to the opening in the component storage container and fitting the opening in the second storage container.

It is preferable for the component storage part to have a ventilation channel in which circulation of air is performed with regard to the component storage container, in which a ventilation tube provided with a ventilation filter is further linked to the ventilation channel.

It is preferable for the component storage container to comprise a component collection orifice in a bottom part that is an end part different from one end at which the opening is provided in the component storage container.

It is preferable for the blood storage part and the component storage part to have a self-sustaining shape.

It is preferable for the first cap to further includes a decreasing-diameter part which is configured so that a lower face of the first cap is formed to be indented to a top side and gradually reduces in diameter from a lower end side of the first cap towards an upper end; the component outlet channel to be connected to an upper end part of the decreasing-diameter part; and a face of the decreasing-diameter part extending from a connecting portion between the component outlet channel and the decreasing-diameter part towards a bottom end side of the first cap is configured by a convex curved face that is convex downwards.

It is preferable for a cross-section in a height direction of the decreasing-diameter part to be configured by an arc-shaped curve.

It is preferable for a cross-section in a direction orthogonal to the height direction of the decreasing-diameter part to have a circular shape.

It is preferable for an angle of an upper end part of the decreasing-diameter part to be 10° to 60°.

The present invention relates to a method for preparing platelet-rich plasma performed using the apparatus for separating and storing blood components, the method comprising: a blood introduction step of introducing blood into the blood storage container through the blood inlet channel; a first separation step of separating the blood introduced into the blood storage container in the blood introduction step into a component containing plasma and platelets, and another component; a transfer step of transferring aseptically and hermetically the component containing plasma and platelets that was separated in the first separation step to the component storage part via the linking tube, by causing the blood storage container to deform as if being crushed by pressurizing; a second separation step of separating the component containing plasma and platelets that was transferred to the component storage part in the transfer step into an upper layer containing plasma and a lower layer containing platelets; and a concentration step of concentrating platelets contained in the lower layer by sending back the upper layer that was separated in the second separation step to the blood storage container via the linking tube by returning back to original a shape of the blood storage container.

### Effects of the Invention

According to the present invention, it is possible to provide a device for separating and storing blood components and a method for preparing platelet-rich plasma that can separate and store platelet-rich plasma more easily and aseptically.

### BRIEF DESCRIPTION OF THE DRAWINGS

FIG. 1 is a drawing showing an apparatus for separating and storing blood components according to a first embodiment of the present invention;
FIG. 2 is a partial enlarged view of FIG. 1;
FIG. 3 is a drawing showing a procedure for performing separation and storing of platelet-rich plasma using the apparatus for separating and storing blood components according to the first embodiment of the present invention;
FIG. 4 is a drawing showing a blood introduction step in a blood component separation and storing operation using the apparatus for separating and storing blood components according to the first embodiment of the present invention;
FIG. 5 is a drawing showing a state of containing a blood component separating and storing container in a centrifuge holder, in a separation step of the blood component separation and storing operation using the apparatus for separating and storing blood components according to the first embodiment of the present invention;
FIG. 6 is a drawing showing a state of separating blood components in a first separation step in the blood component separation and storing operation using the apparatus for separating and storing blood components according to the first embodiment of the present invention;
FIG. 7 is a drawing showing a state of transferring blood components to a component storage part, in a transfer step of the blood component separation and storing operation using the apparatus for separating and storing blood components according to the first embodiment of the present invention;
FIG. 8 is a drawing showing a state of separating blood components in a second separation step of the blood component separation and storing operation using the apparatus for separating and storing blood components according to the first embodiment of the present invention;
FIG. 9 is a drawing showing a state of sending back blood components to a blood storage vessel, in a concentration step of the blood component separation and storing operation using the apparatus for separating and storing blood components according to the first embodiment of the present invention;
FIG. 10 is a perspective cross-sectional view of the blood storage container used in an apparatus for separating storing blood components according to a second embodiment of the present invention;
FIG. 11 is a cross-sectional view of a blood storage container used in the apparatus for separating and storing blood components according to the second embodiment of the present invention;
FIG. 12 is a perspective view of a first cap of the blood storage container used in the apparatus for separating and storing blood components according to the second embodiment of the present invention;
FIG. 13 is a partial cross-sectional perspective view of the first cap of the blood storage container used in the apparatus for separating and storing blood components according to the second embodiment of the present invention;
FIG. 14 is a drawing showing an apparatus for separating and storing blood components according to a third embodiment of the present invention; and
FIG. 15 is a drawing showing a state of separating a blood storage part and component storage part of the apparatus for separating and storing blood components shown in FIG. 14.

### PREFERRED MODE FOR CARRYING OUT THE INVENTION

Hereinafter, an apparatus for separating and storing blood components of the present invention will be explained while referencing the drawings, based on a preferred embodiment thereof.

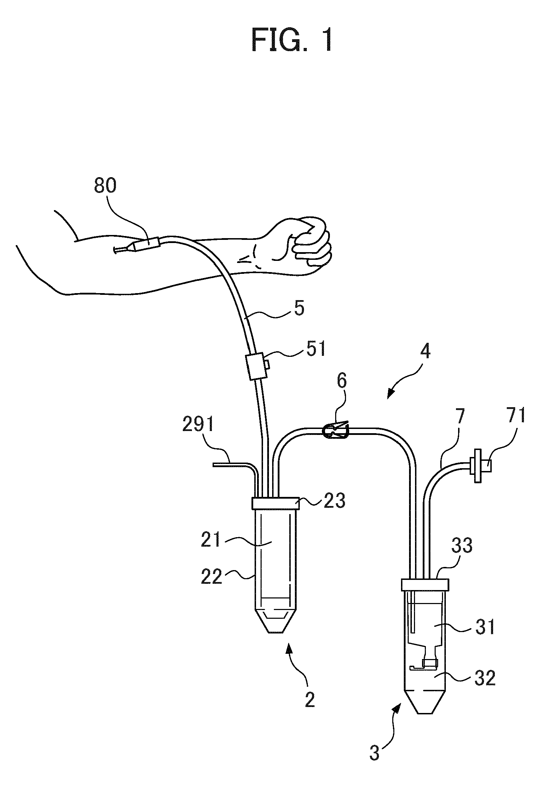

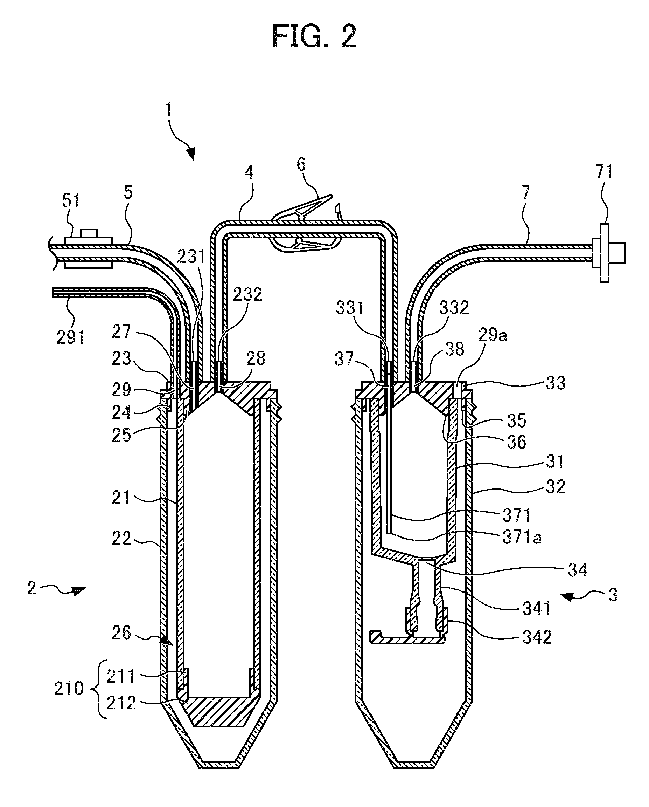

An apparatus 1 for separating and storing blood component of the first embodiment, as shown in FIG. 1 and FIG. 2, is provided with a blood storage part 2 for storing blood, a component storage part 3 for storing a part of the components of the blood stored in the blood storage part, a linking tube 4 that aseptically links the blood storage part 2 and the component storage part 3, and a clamp 6 serving as an closing-off device provided to the linking tube 4.

The term "blood" used herein indicates whole blood including hemocytes (erythrocytes, leucocytes, platelets) and plasma (serum) as a liquid component.

The blood storage part 2, as shown in FIG. 2, is provided with a blood storage container 21, a first storage container 22 for containing the blood storage container 21, and a first cap 23 connected to the blood storage container 21 and the first storage container 22.

The blood storage container 21, as shown in FIG. 2, has a longitudinal cylindrical shape, and is formed from a material whose side faces are flexible. Furthermore, a cross-section in a radial direction of the blood storage container 21 has an elliptical shape.

A side face of the blood storage container 21 is preferably composed of material that is transparent from a viewpoint in which blood stored inside the blood storage container 21 is visible. For the flexible material it is possible to use polyvinyl chloride, polyethylene, polypropylene, polyurethane, silicone, ethylene-vinyl acetate copolymer resin, synthetic rubber, and soft synthetic resin such as various types of elastomer. An upper end which is one end of the blood storage container 21 has an opening, and is hermetically sealed by fitting the first cap 23. A bottom cap 210 is fitted to a lower end portion which is the other end of the blood storage container 21, and is joined by an adhesive.

The bottom cap 210 is provided with a cylindrical fitting part 211 and a bottom part 212 contiguous with this fitting part. A cross-section in a radial direction of the fitting part 211 is circular shape, and an external diameter thereof is approximately the same as an internal diameter of the blood storage container 21. With regard to the bottom cap 210, the fitting part 211 fits a lower end part of the blood storage container 21. The diameter of the bottom part 212 gradually decreases in a downward direction, after which a bottom face thereof has a flat shape.

The first storage container 22, as shown in FIG. 2, has a cylindrical shape longitudinally similar to the blood storage container 21, and is configured such that its diameter and height are both larger than the blood storage container 21. An upper end which is one end of the first storage container 22 also has an opening, and can be hermetically sealed by fitting the first cap 23. In a vicinity of a lower end portion which is another end of the first storage container 22, the diameter of the first storage container 22 gradually decreases in a downward direction, after which it has an upper-lower reversed conical shape with a flat bottom face on the head thereof.

The first storage container 22 preferably is formed from material that is transparent from a viewpoint in which blood stored inside the blood storage container 21 is visible, and is preferably formed from material having relatively large rigidity. With regard to the material of which the first storage container 22 is composed, specifically, hard synthetic resin such as polycarbonate, polyethylene, polypropylene, polyester, polymethylpentene, methacryl, ABS resin (acrylonitrile butadiene styrene copolymer), PET resin (polyethylene-telephthalate) and polyvinyl chloride may be cited. It should be noted that the hard synthetic resin refers to synthetic resin having hardness of an extent that does not easily deform due to a pressure variation of an internal space of a container due to injection of fluid to be described later.

A screw ridge is formed on an outer side face in a vicinity of the upper end of the first storage container 22, and a cover cap (not shown in the drawings) having a screw trough corresponding to the screw ridge can be screwed on. By screwing the cover cap onto the first storage container 22, it is possible to prevent the first cap 23 from coming off the first storage container 22 during a blood component separation and storing operation, as described later.

The first cap 23 has a flat face with a circular shape, and its diameter is approximately the same as the outer diameter of the first storage container 22 (refer to FIG. 2). On a lower face that is one face of the first cap 23, a first connection part 24 that can be connected to the first storage container 22, and a second connection part 25 that is arranged on an inner side of the first connection part 24 and can be connected to the blood storage container 21 are formed. The first connection part 24 and the second connection part 25 have shapes that protrude in a downward direction.

An outer diameter of the first connection part 24 is approximately the same as an inner diameter of the opening of the first storage container 22, and the opening vicinity of the first storage container 22 can be fitted to an outer peripheral face of the first connection part 24. An outer diameter of the second connection part 25 is approximately the same as an inner diameter of the opening of the blood storage container 21, the opening vicinity of the blood storage container 21 can be fitted to an outer peripheral face of the second connection part 25.

That is, in the first embodiment, the blood storage container 21 and the first storage container 22 that contains the blood storage container 21 are both configured to be fitted to the first cap 23. The first storage container 22 is hermetically sealed by being fitted to the first cap 23. Furthermore, in this way, a pressure regulation space 26, which is a space independent of an inner space of the blood storage container 21, is formed between an outer side of the blood storage container 21 and an inner side of the first storage container 22.

A hermetically sealing member (not shown in the drawings) for improving sealing of the pressure regulation space 26 is interposed between the outer peripheral face of the first connection part 24 and an inner peripheral face in the vicinity of the opening of the first storage container 22. In this way, it is possible to ensure a hermetically sealed structure with more certainty for the pressure regulation space 26.

As a hermetically sealing member, it is possible to use, for example, a ring shaped member formed from silicon rubber. It should be noted that the hermetically sealing member may have a configuration which can cap the outer peripheral face of the first connection part 24; material is not limited to the abovementioned silicon rubber, and it is possible to be used elastic members such as various types of rubber, various types of elastomer, and the like.

Furthermore, the hermetically sealing member may be disposed between an outer peripheral face of the second connection part 25 and an inner peripheral face in the vicinity of the opening of the blood storage container 21.

The blood storage part 2 is provided with a blood inlet channel 27 for introducing blood into the blood storage container 21, and a component outlet channel 28 for extracting a part of the components of the blood from the blood storage container 21. In the first embodiment, the blood inlet channel 27 and the component outlet channel 28 are arranged in the first cap 23, as shown in FIG. 2. The blood inlet channel 27 and the component outlet channel 28 are arranged by forming a through hole penetrating the first cap 23 from an upper face thereof to a lower face.

It should be noted that the shape of a portion facing the inner space of the blood storage container 21, with regard to the lower face of the first cap 23, as shown in FIG. 2, is preferably formed to have a gradually decreasing diameter in an upward direction. In addition, a preferable configuration is one in which the component outlet channel 28, which is a through hole in a top part thereof, is provided. With the abovementioned configuration, when the blood component that is separated inside the blood storage container 21 is extracted to the component storage container 31, it is possible to draw out the blood component without waste.

A blood drawing tube 5 serving as a blood inlet tube is connected to the blood inlet channel 27, and blood drawn from a subject passes through the blood drawing tube 5 and the blood inlet channel 27, to be stored aseptically in the blood storage part 2 (the blood storage container 21). The blood inlet channel 27 and the blood drawing tube 5 are connected by engaging an end part of the blood drawing tube 5 with a first protruding part 231 arranged on an upper face side in a portion in which the blood inlet channel 27 is formed, in the first cap 23.

The apparatus 1 for separating and storing blood components of the first embodiment further includes a co-injection port 51 serving as an anticoagulant introduction part that is provided to the blood drawing tube 5 and introduces an anticoagulant to the blood storage container 21. As the anticoagulant introduced from the co-injection port 51 to the blood storage container 21, sodium citrate, EDTA, heparin, ACD (Acid Citrate Dextrose Solution), etc. can be exemplified. From the viewpoint of the preservation of platelets in blood before preparation of platelet-rich plasma, or during preparation, it is preferable to introduce sodium nitrate as the anticoagulant from the co-injection port 51 to the blood storage container 21.

The blood storage part 2, as shown in FIG. 2, is provided with an injection hole 29 through which fluid can be injected into the pressure regulation space 26. Furthermore, an injection means (not shown in the drawings) that injects fluid into the pressure regulation space 26 is linked to the injection hole 29. In the first embodiment, the injection hole 29 is arranged in the first cap 23. In detail, the injection hole 29 is a through-hole arranged in the first cap 23, and is formed in an area on an outer side of the second connection part 25 and an inner side of the first connection part 24 in the first cap 23.

One end of a fluid injection tube 291 is connected to the injection hole 29, and an injection means is connected to the other end of the fluid injection tube 291. A pump, syringe, or the like can be used as the injection means.

By injecting fluid from the injection hole 29 by the injection means, the pressure regulation space 26 is pressurized. At this time, since the first storage container 22 forming an outer side of the pressure regulation space 26 is not flexible, the blood storage container 21 is affected by the increased pressure of the pressure regulation space 26 and is deformed as if being crushed. Furthermore, the blood storage container 21 that is deformed as if being crushed by the pressurization of the pressure regulation space 26 can restore to its original shape by depressurizing the pressure regulation space 26 that has been pressurized. In addition, by the closing off or releasing the closing off of the linking tube 4 by the clamp 6 described later, since it is possible to cause a pressure differential to arise between the pressure regulation space 26 and the internal space of the blood storage container 21, the blood storage container 21 can be made to deform. In this way, by deforming the blood storage container 21, it is possible to easily and aseptically extract a part of the components of blood described later from the first storage container 22 to the component storage part 3, to be described later, and send back from the component storage part 3 to the first storage container 22.

The fluid that is injected into the pressure regulation space 26 may be a gas such as the air, or may be a liquid such as water. Furthermore, a gel form may be used as the fluid. In a case of using a fluid such as water or a substance in a gel form as the fluid, the volume of the fluid doesn't changes due to added pressure at time of injection, and this is preferable in that it is possible to accurately comprehend the amount of blood component that is extracted to the component storage part 3.

A linking tube 4, as shown in FIG. 2, aseptically links the blood storage part 2 and the component storage part 3. In the first embodiment, the linking tube 4 is formed from a flexible tube, and links the component outlet channel 28 formed in the first cap 23, and the component inlet channel 37 formed in the second cap 33, to be described later. The linking tube 4 and the component outlet channel 28 are connected by engaging one end of the linking tube 4 to a second protruding part 232 provided on an upper face side of a portion in which the component outlet channel 28 is formed in the first cap 23.

The clamp 6 is provided to the linking tube 4, and blocks the extraction of a part of the components of blood described later from the first storage container 22 to the component storage part 3, and the sending back from the component storage part 3 to the first storage container 22.

The component storage part 3, as shown in FIG. 2, is provided with the component storage container 31, a second storage container 32 that contains the component storage container 31, the second cap 33 that is joined to the component storage container 31 and the second storage container 32, and a metal needle 371 serving as a return pipe linked to the second cap 33.

The component storage container 31 has a longitudinal cylindrical shape, and is formed from material having a flexible side face. A material similar to the abovementioned blood storage container 21 can be used as the flexible material. An upper end, which is one end of the component storage container 31, has an opening, and is hermetically sealed by being connected to the second cap 33. The component storage container 31 includes a component collection orifice 34 provided at a lower end of the component storage container 31, a cylindrical part 341 of cylindrical shape protruding at a lower part of the component collection orifice 34, and a cap body 342 that is installed to the cylindrical part 341.

The component collection orifice 34 is formed at a lower end part, which is the other end of the component storage container 31. This component collection orifice 34 is provided in order to collect blood components stored in the component storage container 31. The component collection orifice 34 is formed by providing a thin area of a thin film form at a part of a bottom face of the component storage container 31.

The cylindrical part 341 is formed in a region formed of the component collection orifice 34, so as to protrude downwards from the bottom face of the component storage container 31. The cylindrical part 341 is a hollow cylindrical shape.

The cap body 342 is installed in the cylindrical part 341. The blood components contained inside the component storage container 31 can be collected by breaking and puncturing the component collection orifice 34 with a puncturing tool such as a syringe needle or the like, after removing the cap body 342. At this time, since the hollow cylindrical part 341 is formed in an area formed at the component collection orifice 34, it is possible to assuredly puncture the component collection orifice 34 with the puncturing tool.

It should be noted that, as shown in FIG. 2, the shape of the bottom face of the component storage container 31 is preferably formed to have a gradually decreasing diameter in a downward direction. In addition, the component collection orifice 34 is preferably arranged at an apex thereof. With the abovementioned configuration, when blood component is collected inside the component storage container 31, it is possible to collect the blood component that is to be collected, without waste.

The second storage container 32 has a longitudinal cylindrical shape, and is configured such that its diameter and height are both larger than the component storage container 31. An upper end which is one end of the second storage container 32 also has an opening, and can be hermetically sealed by fitting the second cap 33. In a vicinity of a lower end portion which is the other end of the second storage container 32, the diameter of the second storage container 32 gradually decreases in a downward direction, after which it has an upper-lower reversed conical shape with a flat bottom face on the head thereof.

In the present first embodiment, the second storage container 32 is formed from material the same as the abovementioned first storage container 22, and the shape and size thereof are the same as the first storage container 22.

A screw ridge, similar to the first storage container 22, is formed on an outer side face in a vicinity of the upper end of the second storage container 32, and a cover cap (not shown in the drawings) having a screw trough corresponding to the screw ridge can be screwed on.

The second cap 33, similar to the first cap 23, has a flat face with a circular shape, and its diameter is approximately the same as the outer diameter of the second storage container 32. On a lower face, which is one face of the second cap 33, a third connection part 35 that can be connected to the second storage container 32, and a fourth connection part 36 that is arranged on an inner side of the third connection part 35 and can be connected to the component storage container 31 are formed. The third connection part 35 and the fourth connection part 36 have shapes that protrude in a downward direction.

An outer diameter of the third connection part 35 is approximately the same as an inner diameter of the opening of the second storage container 32, and the opening vicinity of the second storage container 32 can be fitted to an outer peripheral face of the third connection part 35. An outer diameter of the fourth connection part 36 is approximately the same as an inner diameter of the opening of the component storage container 31, the opening vicinity of the component storage container 31 can be fitted to an outer peripheral face of the fourth connection part 36.

As shown in FIG. 2, a through hole 29a is provided in the second cap 33, but this through hole 29a need not be provided.

That is, in the first embodiment, the component storage container 31 and the second storage container 32 that contains the component storage container 31 are both configured to be fitted to the second cap 33. The component storage container 31 is hermetically sealed by being fitted to the second cap 33, and the second storage container 32 is also hermetically sealed by being fitted to the second cap 33.

To the above second cap 33, a component inlet channel 37 is provided for introducing a part of the components of blood that has been drawn from the blood storage container 21. In the first embodiment, as shown in FIG. 2, the component inlet channel 37 is provided by forming a through hole penetrating the second cap 33 from a top face thereof to a bottom face.

The linking tube 4 is connected to the upper face side of the component inlet channel 37 in the second cap 33, and a part of the components of blood drawn from the blood storage container 21 is stored aseptically within the component storage part 3 (the component storage container 31). The component inlet channel 37 and the linking tube 4 are connected by engaging an end part of the linking tube 4 with a third protruding part 331 arranged on an upper face side in a portion in which the fluid inlet channel 37 is formed, in the second cap 33.

The metal needle 371 is connected to a lower face side of the component inlet channel 37 in the second cap 33. The metal needle 371 is a hollow shape. The metal needle 371 extends from the component inlet channel 37 towards an internal space of the component storage part 3 (component storage container 31). The metal needle 371 is able to send back a part of the components of blood stored in the component storage part 3 to the blood storage container 21.

The metal needle 371 projects from an end part of the component inlet channel 37 at the lower face side of the second cap 33 to the bottom face of the component storage part 3, and extends to a position with a predetermined distance from the bottom face of the internal space of the component storage container 31, as shown in FIG. 2. The metal needle 371 draws a part of the components of blood stored inside of the component storage unit 3 (component storage container 31), upon restoring the blood storage container 21 that was deformed as if being crushed to its original shape.

A ventilation channel 38 for air circulation to and from the component storage container 31 is provided in the component storage part 3, and a ventilation tube 7 provided with a ventilation filter 71 is further linked to the ventilation channel 38. In the first embodiment, the ventilation channel 38 is provided in the second cap 33. In detail, the ventilation channel 38 is a through hole provided in the second cap 33, and is formed in an area on an inner side of the fourth connection part 36 in the second cap 33.

One end of the ventilation tube 7 is connected to the ventilation channel 38, and the ventilation filter 71 is connected to the other end of the ventilation tube 7. The ventilation filter 71 is a filter having a property by which air is passed but fluid is not passed, and bacteria are also not passed. That is, the ventilation filter 71 can aseptically circulate air to and from the inside the component storage container 31, from the linked ventilation channel 38. The ventilation channel 38 and the ventilation tube 7 are connected by engaging an end part of the ventilation tube 7 to a fourth protruding part 332 provided on an upper face side of a portion in which the ventilation channel 38 is formed in the second cap 33.

In the apparatus for separating and storing blood components 1 of the first embodiment having the abovementioned configuration, the blood storage part 2 (the blood storage container 21), the linking tube 4, and the component storage part 3 (the component storage container 31) are aseptically linked, and internal spaces in each of the blood storage container 21, the linking tube 4, and the component storage container 31 are kept in an aseptic state. As a result, by circulating air aseptically to and from the ventilation channel 38, the apparatus for separating and storing blood components 1 of the first embodiment can regulate the internal spaces in each of the blood storage container 21, the linking tube 4, and the component storage container 31, to an arbitrary pressure while aseptically maintaining an aseptic state.

Next, a description will be given concerning a preferable size of each component member in the apparatus for separating and storing blood components 1 of the first embodiment.

A stored amount of blood in the blood storage container 21 is preferably from 5 to 200 ml, and more preferably 5 to 50 ml. The blood storage container 21 specifically has a preferable internal diameter of 10 to 30 mm, and a preferable height thereof is 50 to 150 mm.

The stored amount of blood components in the component storage container 31, from the viewpoint that fluid components separated from the blood stored in the blood storage container 21 are assuredly stored, is preferably 40 to 100% of the blood storage amount in the blood storage container 21.

If the first storage container 22 and the second storage container 32 are of sizes that can respectively contain the blood storage container 21 and the component storage container 31, there is no particular limitation on size, but diameters thereof are preferably 10 to 30 mm, and heights thereof are preferably 50 to 150 mm.

Furthermore, well known centrifuge separating tubes are preferably used as the first storage container 22 and the second storage container 32. By using the well-known centrifuge separating tubes as the first storage container 22 and the second storage container 32, in a centrifuge separation step in a blood component separation operation described below, it is possible to perform the separation operation simply by using a normal centrifuge separator in which the centrifuge separating tube can be used.

When well-known centrifuge separating tubes are used as the first storage container 22 and the second storage container 32, the volume of the centrifuge separating tubes, from the viewpoint of raising general usability in the centrifuge separation process, is preferably 5 to 50 ml.

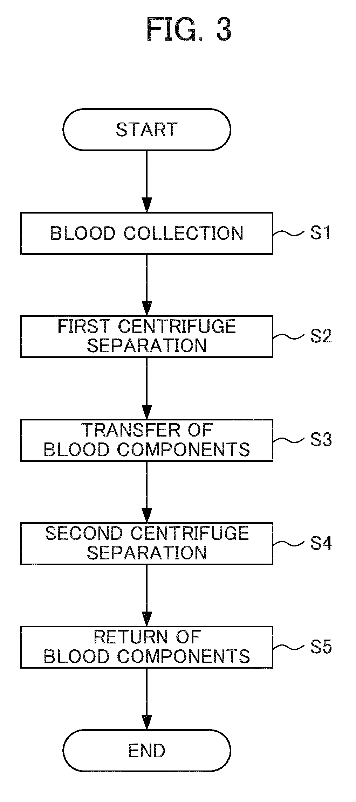

Next, using FIG. 3 to FIG. 9, a description is given concerning one preferred mode of a blood component separation and storing operation (method for preparing platelet-rich plasma) using the apparatus for separating and storing blood components 1 of the first embodiment having the abovementioned configuration.

As shown in FIG. 3, the blood component separation and storing operation of the present mode is generally made up of five steps (S1 to S5).

First, in a blood introduction step S1, blood is introduced to the blood storage container 21 through the blood inlet channel 27.

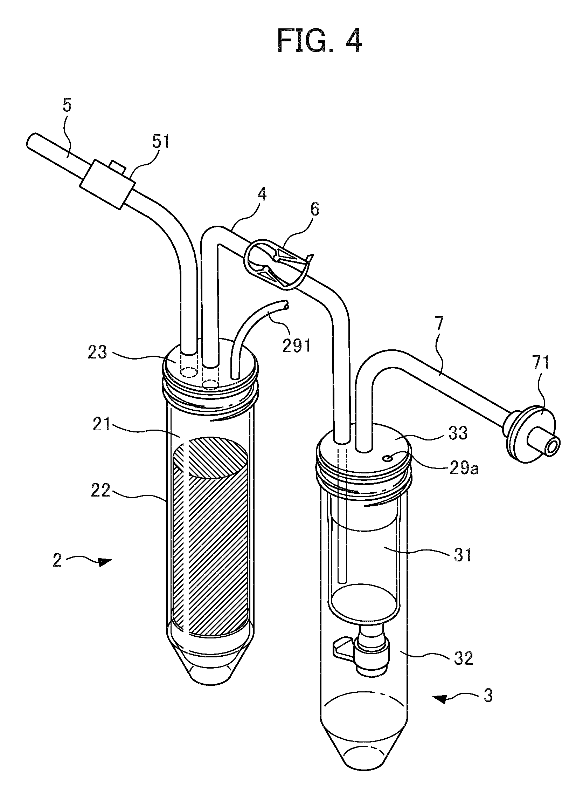

In the blood introduction step S1, as shown in FIG. 1, a blood drawing needle 80 is inserted into a subject (patient), and blood is drawn. At this time, the blood drawn by the blood drawing needle 80 is stored inside a blood storage part 2 (blood storage container 21), via a blood drawing tube 5 (refer to FIG. 4). When the blood is collected, by drawing in air from a ventilation filter 71 located at an extremity of a ventilation tube 7 provided in a component storage part 3, a negative pressure is applied inside a component storage container 31 and the blood storage container 21 communicating thereto, and it is possible to simply draw blood into the blood storage part 2. Furthermore, a non-return valve (not shown in the drawing) for preventing blood from back-flowing to the subject while blood is being drawn may be provided in the blood drawing tube 5.

In the blood introduction step S1, consideration is given to the physical condition of a patient when blood is taken, a required amount is collected, and the step is completed. The required amount referred to here is approximately 15 to 20 ml when there is no problem with the patient's body size and condition. In addition, after the blood introduction step S1, the path of the linking tube 4 is closed using the clamp 6, so that the blood drawn to the blood storage unit 2 does not flow into the component storage part 3 (refer to FIG. 4).

After blood collection, the blood needle 80 is withdrawn from the subject (patient), a part of the blood drawing tube 5, which connects the blood drawing needle 80 and the blood storage part 2, is melt-cut, and at the same time the melt-cut end is melted. A melting cutter (not shown in the drawings) referred to as a sealer can be used in melt-cutting the drawing tube 5.

It should be noted that, prior to blood collection, it is preferable to introduce an anticoagulant from the co-injection port 51 into the blood storage container 21. Prior to blood collection, the anticoagulant is introduced into the blood storage container 21 from the co-injection port 51, and the blood and anticoagulant are mixed, whereby it becomes difficult for the blood (blood components) to coagulate during the preparation of platelet-rich plasma. In addition, concurrently with blood collection, or after blood collection, the anticoagulant may be introduced into the blood storage container 21 from the co-injection port 51.

In the first separation step S2, the blood that was introduced into the blood storage container 21 in the blood introduction step S1 is separated into a component containing plasma and platelets, and other components.

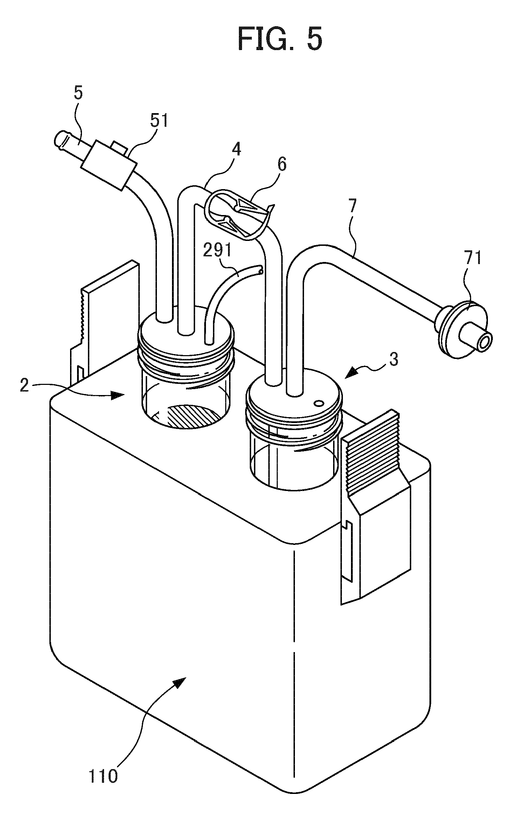

A centrifuge separator is applied to the blood storage part 2, which has been separated from the subject whose blood was collected, together with the component storage part 3, the linking tube 4, the ventilation tube 7, and the like. At this time, with the blood storage part 2 (the first storage container 22) and the component storage part 3 (the second storage container 32), in a case of using a well-known centrifuge separation tube, as shown in FIG. 5, the blood storage part 2 and the component storage part 3 are inserted in a centrifuge holder 110 used for the centrifuge separator, and it is possible to perform centrifuge separation simply. The linking tube 4 is maintained in a state in which a path is closed by the clamp 6.

A condition of the centrifuge separation with respect to the blood storage part 2 is that a setting is made according to the amount of blood stored and the type of components separated, and a setting is made, for example, to 900 g × 10 minutes at room temperature (20 to 30°C).

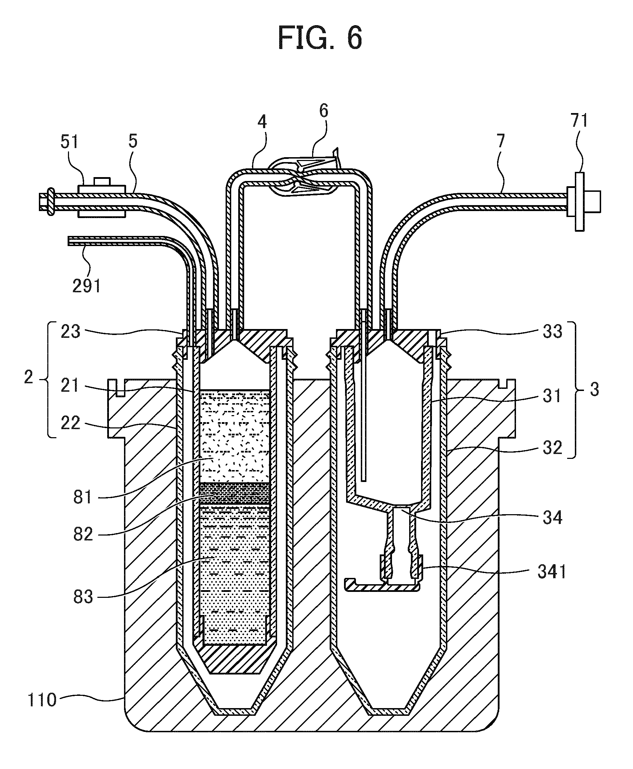

The blood that has undergone centrifuge separation is generally separated and fractioned into the three layers of a plasma layer 81, a buffy coat layer 82 mainly containing white blood cells and platelets, and a red blood cell layer 83, inside the blood storage part 2 (the blood storage container 21), as shown in FIG. 6. In FIG. 6, although the plasma layer 81, buffy coat layer 82 and red blood cells layer 83 are clearly fractionated, since the conditions of the above-mentioned centrifuge separation are comparatively mild conditions, the boundaries between these three layers are not necessarily clear. The components including plasma and platelets separated in the first separation step S2 are the plasma layer 81 and the buffy coat layer 82, and the other component is the red blood cell layer 83.

In the transfer step S3, the component including plasma and platelets that was separated in the first separation step S2 (plasma layer 81 and buffy coat layer 82) is aseptically transferred to the component storage part 3 via the linking tube 4, by causing the blood storage container 21 to deform so if being crushed by pressurizing.

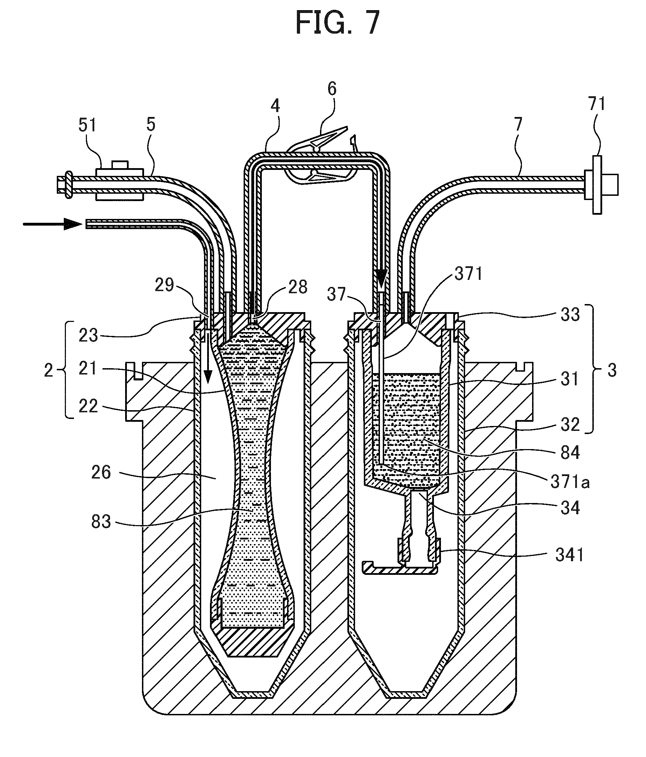

In other words, as shown in FIG. 7, in the transfer step S3, the component including plasma and platelets (plasma layer 81 and buffy coat layer 82) that was separated inside the blood storage container 21 is extracted to the component storage container 31 in the component storage part 3.

When the plasma layer 81 and buffy coat layer 82 separated inside the blood storage container 21 are extracted to the component storage container 31, first the closure of the path of the linking tube 4 by the clamp 6 is released. Next, as shown in FIG. 7, the pressure regulation space 26 is pressurized by injecting fluid from the injection hole 29 to the pressure regulation space 26 formed between the blood storage container 21 and the first storage container 22 in the blood storage part 2. A pump, syringe, or the like can be used as the injection means for injecting the fluid, and a gas such as the air, a fluid such as water, or a substance in a gel form can be used as the fluid. Here, since the first storage container 22 forming an outer side of the pressure regulation space 26 is relatively rigid, the blood storage container 21, which has flexibility, is affected by increased pressure of the pressure regulation space 26 and deforms as if being crushed; the plasma layer 81 and buffy coat layer 82, which have been separated, are extracted via the linking tube 4 from the component outlet channel 28, and are drawn into the component storage part 3 (the component storage container 31) through the component inlet channel 37 and metal needle 371.

In the transfer step S3, along with pressurization of the pressure regulation space 26 as described above, air may be drawn in from the ventilation filter 71 positioned at an extremity of the ventilation tube 7 provided in the component storage part 3. By drawing air from the ventilation filter 71, a negative pressure is applied inside the component storage container 31 and the linking tube 4 communicating therewith, and it is possible to draw the plasma layer 81 and buffy coat layer 82 from the blood storage container 21 to the component storage container 31 more easily.

It should be noted that the plasma layer 81 and buffy coat layer 82, after being transferred to the component storage part 3 in the transfer step S3, are mixed to form a mixed liquid 84 (refer to FIG. 7).

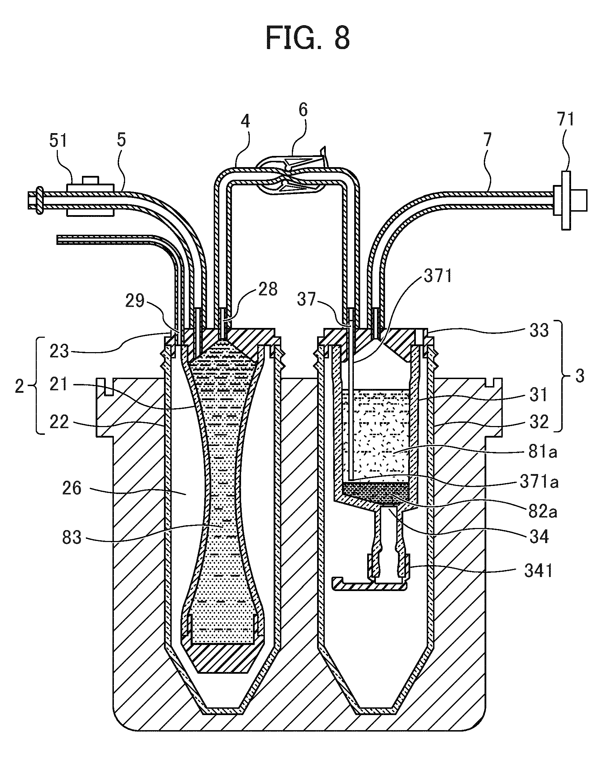

In the transfer step S3, after transferring the component including plasma and platelets (plasma layer 81 and buffy coat layer 82) to the component storage part 3, the path of the linking tube 4 is closed again using the clamp 6 (refer to FIG. 8). After the path of the linking tube 4 has been closed using the clamp 6, pressurizing of the pressure regulation space 26 is released. By the path of the linking tube 4 being closed using the tube 6, it is possible to maintain a state in which the blood storage container 21 deforms, even when releasing the pressurizing of the pressure regulation space 26. By maintaining a state in which the blood storage container 21 is deforming, it is possible to prevent sending back to the blood storage part 2 side of the blood components transferred to the component storage part 3 at this stage.

After the transfer step S3, a leading end 371a of the metal needle 371 is at a position immersed in the component including plasma and platelets that is stored in the component storage part 3 (component storage container 31).

In the second separation step S4, the component including plasma and platelets (mixed liquid 84) that was transferred to the component storage part 3 in the transfer step S3 is separated into an upper layer containing plasma, and a lower layer containing platelets.

In the second separation step S4, a centrifuge separator is applied to the component storage part 3, together with the blood storage part 2, the linking tube 4, the ventilation tube 7, and the like. At this time, with the blood storage part 2 (the first storage container 22) and the component storage part 3 (the second storage container 32), in a case of using a well-known centrifuge separation tube, as shown in FIG. 5, the blood storage part 2 and the component storage part 3 are inserted in the centrifuge holder 110 used for the centrifuge separator similarly to the centrifuge separation in the aforementioned first separation step S2, and it is possible to perform centrifuge separation simply. The linking tube 4 is maintained in a state in which a path is closed by the clamp 6 (refer to FIG. 8).

A condition of the centrifuge separation with respect to the blood storage part 2 is that a setting is made according to the amount of blood stored, and a setting is made, for example, to 2000 g × 10 minutes at room temperature (20 to 30°C).

The blood components that have undergone centrifuge separation is generally separated and fractioned into the two layers of a plasma layer 81a (upper layer containing plasma) and a platelet-rich plasma layer (lower layer containing platelets and white blood cells), inside the component storage part 3 (the component storage container 31), as shown in FIG. 8. It should be noted that blood cells (mainly red blood cells mixed in the transfer step S3) settle by centrifuge separation at the lower part of the platelet-rich plasma layer 82a, and adhere to the bottom face of the component storage container 31. In the second separation step S4, since centrifuge separation is performed at conditions that enhance the centrifugal acceleration compared to the first separation step S2, the boundary between the plasma layer 81a and the platelet-rich plasma layer 82a shown in FIG. 7 becomes clearer than the boundaries of the three layers shown in FIG. 6.

The leading end 371a of the metal needle 371 after the second separation step S4 is positioned somewhat more to a plasma layer 81a side than the boundary between the plasma layer 81a and the platelet-rich plasma layer 82a, as shown in FIG. 8.

Generally, 2 mL of platelet-rich plasma can be prepared from 20 mL of blood. Therefore, when specifically introducing 20 mL of blood to the blood storage part 2 in the blood introduction step S1, the leading end 371a of the metal needle 371 is arranged so that the volume of the component storage container 31 more in the bottom face direction than the leading end 371a is 2 mL. By the leading end 371a being positioned somewhat more to the plasma layer 81a side than the boundary between the plasma layer 81 and the platelet-rich plasma layer 82a, it is possible to efficiently remove the plasma layer 81a in the concentration step S5 described later, and thus the reduction in the amount of platelet-rich plasma ultimately prepared can be curbed.

In the concentration step S5, by restoring to the original shape of the blood storage container 21, the upper layer (plasma layer 81a) separated in the second separation step S4 is sent back to the blood storage container 21 via the linking tube 4, whereby the platelets contained in the lower layer (platelet-rich plasma 82a) are concentrated.

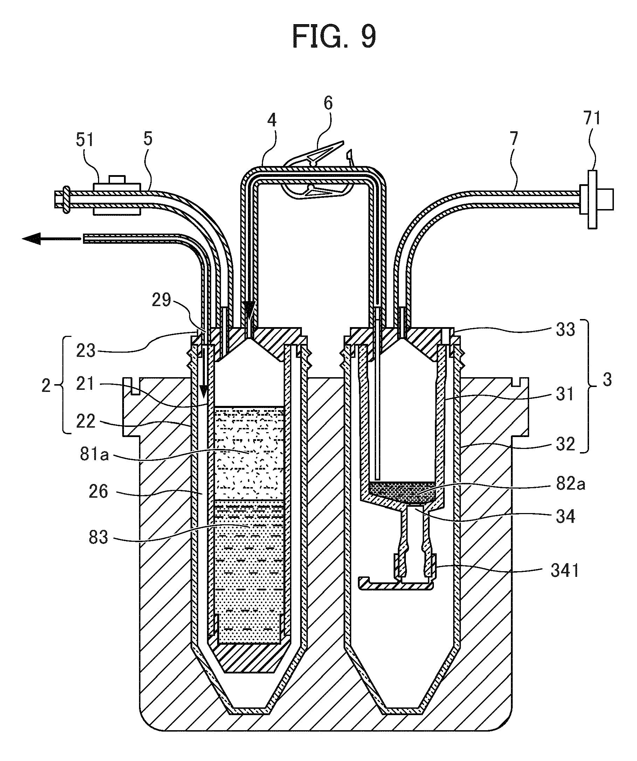

Upon sending back the plasma layer 81a that was separated inside of the component storage part 3 (component storage container 31) to the blood storage container 21, first the closure of the path of the linking tube 4 by the clamp 6 is released (refer to FIG. 9). When releasing the closure of the path of the linking tube 4 by the clamp 6, the shape of the blood storage container 21 having flexibility that had been deformed as if being crushed returns to original. By the shape of the blood storage container 21 returning to original, it is possible to draw in a part of the blood components (plasma layer 81a) from the leading end 371a of the metal needle 371 and send back to the blood storage container 21. By the plasma layer 81a being sent back to the blood storage container 21 in this way, the platelet-rich plasma 82a is concentrated, whereby platelet-rich plasma is prepared.

As mentioned previously, the leading end 371a of the metal needle 371 is positioned somewhat more to the plasma layer 81a side than the boundary between the plasma layer 81a and the platelet-rich plasma layer 82a; therefore, the metal needle 371 can selectively draw in the plasma layer 81a and send back to the blood storage container 21.

It should be noted that, in the concentration step S5, the sending back of blood components to the blood storage container 21 may be aided by sending air to the interior of the component storage container 31 from the ventilation tube 7.

As shown in FIG. 9, after the concentration step S5, the platelet-rich plasma (platelet-rich plasma layer 82a) is stored inside the component storage part 3 (component storage container 31), and mainly red blood cells (red blood cell layer 83) and plasma (plasma layer 81a) are stored inside the blood storage part 2 (first storage container 22).

After the platelet-rich plasma (platelet-rich plasma layer 82a) has been concentrated, the linking tube 4 and the ventilation tube 7 are melt-cut and melted. With regard to this melt-cut and melting, a method can be used that is the same as that of melt-cutting and melting the drawing tube 5 before the first separation step S2.

The prepared platelet-rich plasma (platelet-rich plasma layer 82a) can be collected by removing the cap body 342, and breaking and puncturing the component collection orifice 34 with a puncturing tool such as a syringe needle. In addition, as required, blood cells settled in the second separation step S4 prior to collecting platelet-rich plasma may be collected to improve the purity of the platelet-rich plasma.

According to the apparatus 1 for separating and storing blood components of the first embodiment having the abovementioned configuration, since the blood storage part 2 has the blood storage container 21 that has a cylindrical form and is flexible, and further has the metal needle 371 that extends to the interior space of the component storage part 3 and is able to send back a part of the components of blood stored in the component storage part 3 to the blood storage container 21, it is possible to separate and concentrate platelet-rich plasma more simply. Furthermore, since the blood storage part 2, the component storage part 3, and the linking tube 4 that links these are connected aseptically, the blood or platelet-rich plasma is not affected by the external environment, the danger of the prepared platelet-rich plasma being contaminated by microbes or the like is low, and it is possible to prepare the platelet-rich plasma with a high degree of safety.

Furthermore, by the blood storage container 21 having a longitudinal cylindrical shape, even with a relatively small amount of blood collected, it is possible to easily separate the blood components, and it is possible to easily extract the separated components from the blood storage container 21. The ability to easily separate and store blood components even with a relatively small amount of blood in this way is particularly effective when preparing platelet-rich plasma from the blood of a subject for which the amount of blood that can be drawn is limited. Here, a relatively small amount of blood indicates, in specific terms, a blood amount of approximately from 5 to 50 ml.

Furthermore, by forming the pressure regulation space between the blood storage container 21 and the first storage container 22 in the blood storage part 2 and the injection hole 29 for injecting the fluid into the pressure regulation space, it is possible to pressurize the pressure regulation space 26 and to deform the blood storage container 21 as if it were being crushed. In this way, by deforming the blood storage container 21 by pressurizing the pressure regulation space 26, it is possible to easily and aseptically draw the blood components to the component storage part 3.

In addition, a force trying to restore to the original shape acts on the blood storage container 21 that was deformed as if being crushed by pressurizing the pressure regulation space 26. When the blood storage container 21 restores to the original shape from a form deformed as if being crushed, the interior of the blood storage container 21 becomes negative pressure, whereby the blood components stored in the interior of the component storage part 3 are drawn from the leading end 371a of the metal needle 371.

In addition, since the metal needle 371 serving as a return pipe is configured by a hard member (metal), the position of the leading end 371a of the metal needle 371 will not change from deformation of the metal needle 371. Therefore, according to the apparatus 1 for separating and storing blood components of the first embodiment, it is possible to accurately collect a part of the components of blood stored in the component storage part 3 and send back to the blood storage container 21.

Furthermore, by the first storage container in the blood storage part 2 and the second storage container 32 in the component storage part 3 having a longitudinal cylindrical shape, postural maintenance of the apparatus for separating and storing blood components 1 that includes the blood storage part 2 and the component storage part 3 becomes easy, and operability of the blood component separation container is improved. Furthermore, with the first storage container 22 and the second storage container 32 having the same shape and having a prescribed shape, storage is possible in an ordinarily used centrifuge holder, without using a special support device, application is possible to the centrifuge separator, and the general applicability of the apparatus for separating and storing blood components 1 is improved. In addition, by containing the blood storage part 2 and the component storage part 3 in the centrifuge holder, since the disposition of the apparatus for separating and storing blood components 1 can be maintained, it is possible to use the centrifuge holder as an operational support device that can be commonly used in a series of steps extending from the blood introduction step S1 to the concentration step S5. The prescribed shape refers to cases of, for example, a shape similar to a 50 ml commercially available centrifuge settling tube.

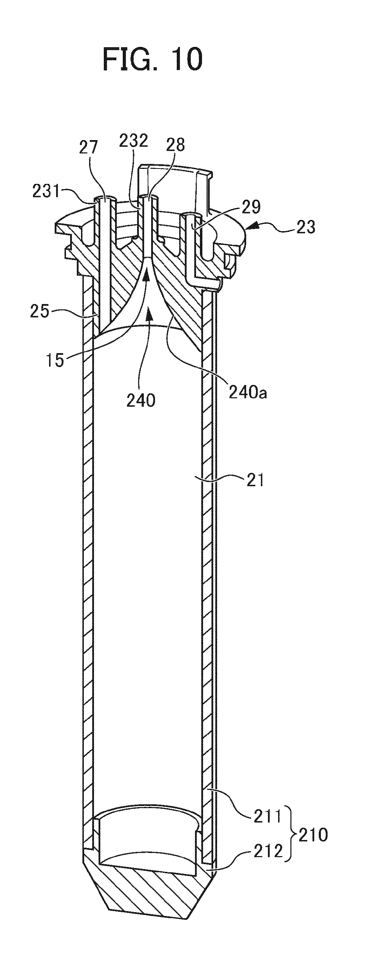

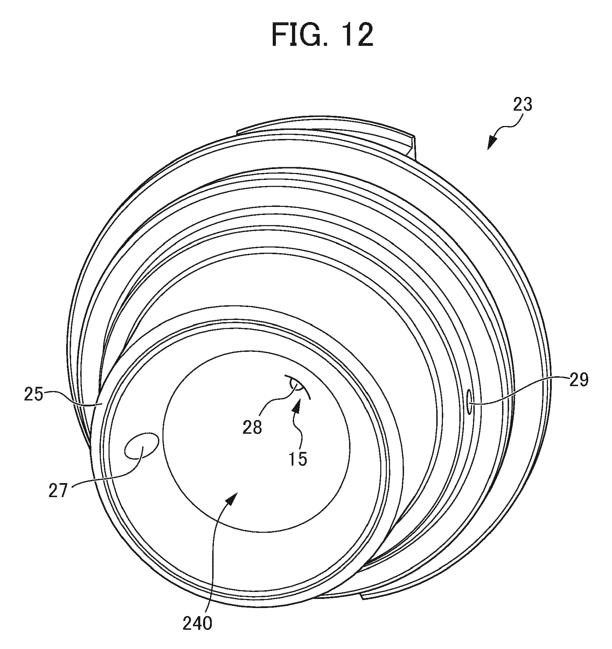

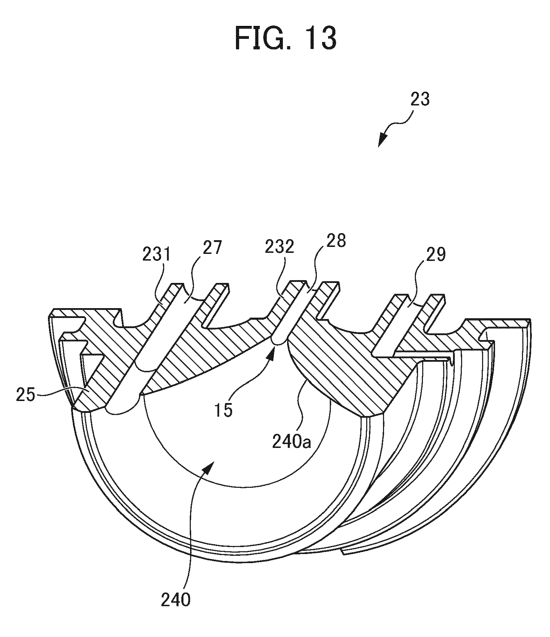

Next, an apparatus for separating and storing blood components according to a second embodiment of the present invention will be explained while referencing FIGS. 10 to 13. The apparatus for separating and storing blood components of the second embodiment mainly differs from the first embodiment in the shape of the lower face of the first cap 23.

It should be noted that, in the explanation of the second and later embodiments, the same reference numbers are assigned for the same constituent elements, and explanations thereof will be omitted or abbreviated.

In the second embodiment, a decreasing-diameter part 240 is formed in the lower face of the first cap 23. The decreasing-diameter part 240 is formed by the lower face of the first cap 23 indenting to an upper face side. The diameter of the concave shape of this decreasing-diameter part 240 gradually becomes smaller from the lower end side of the first cap 23 towards the upper end side (decreasing diameter). Then, at the upper end part of the decreasing-diameter part 240, the component outlet channel 28 is connected. In addition, the cross-section in a direction orthogonal to the height direction of the decreasing-diameter part 240 is a circular shape. Furthermore, a face of the decreasing-diameter part 240 extending from a connecting portion 15 between the component outlet channel 28 and the decreasing-diameter part 240 towards a lower end side of the first cap 23 is configured by a convex curved face 240a that is convex downwards. In other words, the decreasing-diameter part 240 is configured by one convex curved face 240a.

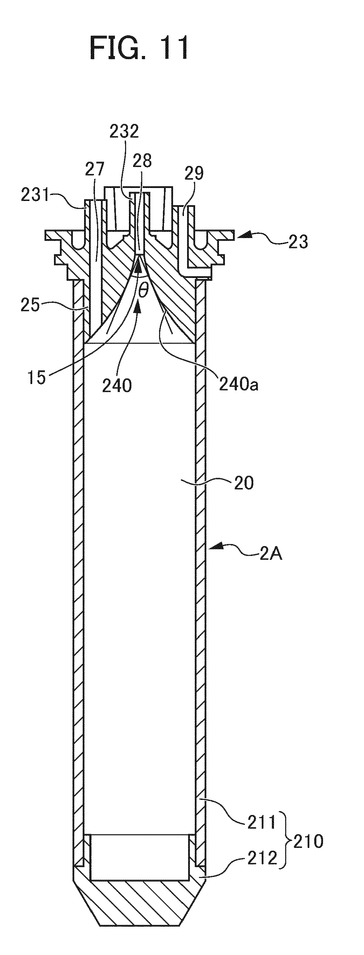

The upper end part of the decreasing-diameter part 240, i.e. an opening angle θ of the decreasing-diameter part 240 at the connecting portion 15 between the reduced-diameter 240 and component outlet channel 28 (refer to FIG. 11) is preferably 10° to 60°, more preferably 15° to 40°, and most preferably 20°, from the viewpoint of performing separation of blood components with higher purity.

According to the apparatus for separating and storing blood components according to the second embodiment, the following such effects are exerted in addition to exerting similar effects as the first embodiment.

The decreasing-diameter part 240 is formed at the lower face of the first cap 23, and the component outlet channel 28 is connected to an upper end part of this decreasing-diameter part 240. It is thereby possible to thicken the thickness of the layer of blood components by gradually thinning the path of the blood components through the decreasing-diameter part 240. Consequently, even when transferring the buffy coat layer 82 of thin thickness, for example, since it can be transferred in a state with a thickened thickness in the decreasing-diameter part 240, the transfer efficiency of the desired blood component can be improved.

In addition, a face that extends from the connecting portion 15 between the decreasing-diameter part 240 and the component outlet channel 28 towards the lower end part of the first cap 23 is configured by the convex curved face 240a that is convex downwards. Since the blood component moving in the upwards direction through the decreasing-diameter part 240 can thereby be transferred to the component outlet channel 28 without being disturbed, the layer of separated blood component is prevented from being disturbed and mixing. Consequently, the transfer efficiency of blood component can be further improved.

In addition, it is configured so that the cross-section of the decreasing-diameter part 240 in a direction orthogonal to the height direction is configured in a circular shape. Since it is thereby possible to configure the decreasing-diameter part 240 by one convex curved face 240a, it can be made more difficult for the layer of blood component being sent to be disturbed.

A cross-section in the height direction of the decreasing-diameter part 240 is configured by an arc-shaped curve. It is thereby possible to realize the first cap 23 that can transfer in a state thickening the layer of the blood component by providing the decreasing-diameter part 230, without increasing the height of the first cap 23 too much. Consequently, an apparatus for separating and storing blood components able to transfer blood components with high purity can be made compact.

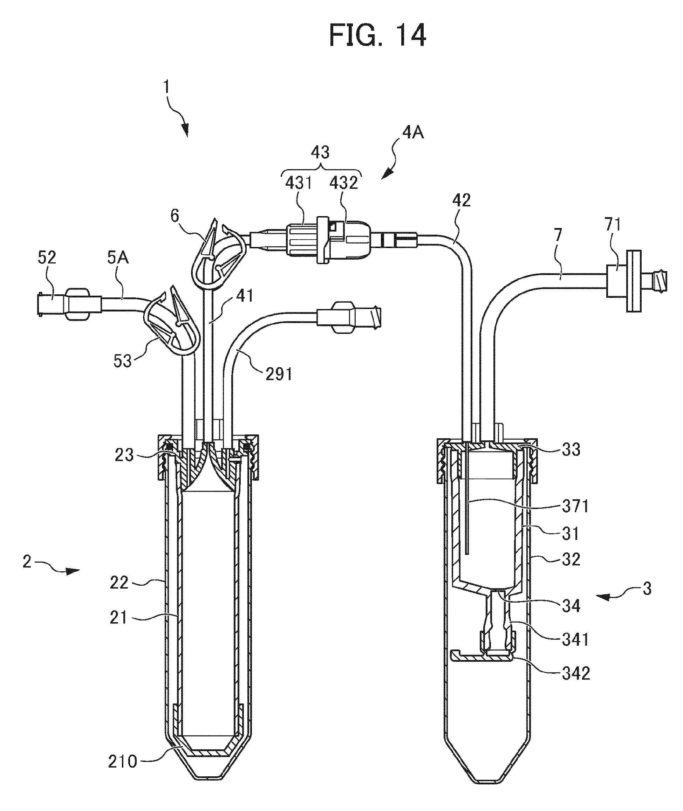

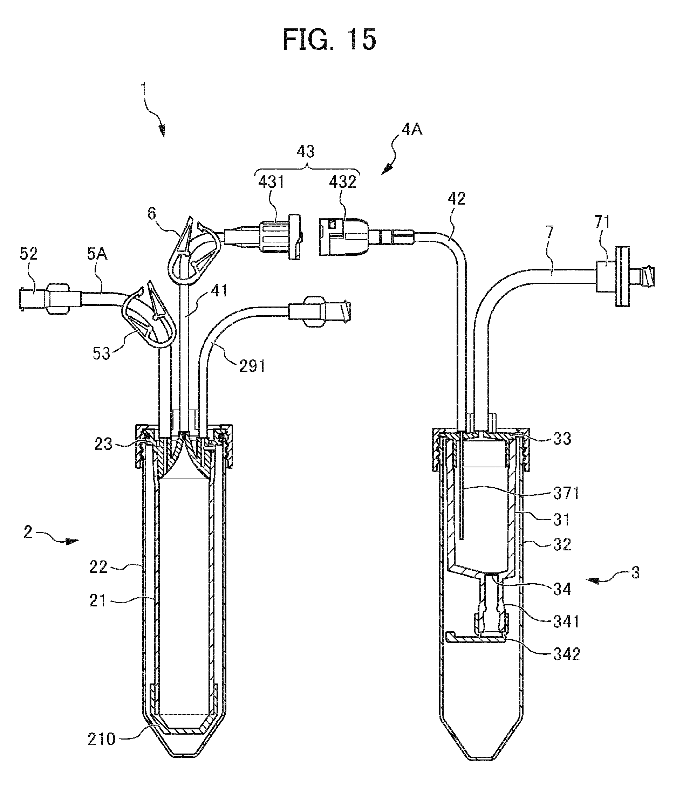

Next, an apparatus 1 for separating and storing blood components according to a third embodiment of the present invention will be explained while referencing FIGS. 14 and 15. The apparatus 1 for separating and storing blood components of the third embodiment differs from the second embodiment in the configurations of a linking tube 4A and blood introduction tube 5A.

In the apparatus 1 for separating and storing blood components of the third embodiment, the linking tube 4A includes a blood storage part-side tube 41, a component storage part-side tube 42 and a connector 43.

The blood storage part-side tube 41 has one end side connected to the blood storage part 2. The component storage part-side tube 42 has one end side connected to the component storage part 3.

The connector 43 detachably links the blood storage part-side tube 41 and the component storage part-side tube 42. More specifically, the connector 43 includes a first connector part 431 that is installed to the other end side of the blood storage part-side tube 41, and a second connector part 432 that is installed to the other end side of the component storage part-side tube 42 and makes detachable to the first connector part 431.

In the third embodiment, the clamp 6 is installed to the blood storage part-side tube 41.

In addition, with the apparatus 1 for separating and storing blood components of the third embodiment, a blood inlet port 52 and clamp 53 are provided to a blood inlet tube 5A.

The blood inlet port 52 is provided at a base end side of the blood inlet tube 5A to which the leading end side thereof is connected to the blood inlet channel. A syringe or the like is connected to this blood inlet port 52.

The clamp 53 is used in the case of closing the path of the blood inlet tube 5A.

In the apparatus 1 for separating and storing blood components of the third embodiment, the blood introduction step S1 and first separation step S2 are performed in a state in which the blood storage part 2 and component storage part 3 are separated by the connector 43.

In the blood introduction step S1, blood is introduced to the blood storage part 2 in a separated state. In the blood introduction step S1, the path of the blood storage part-side tube 41 is closed by the clamp 6. In addition, the blood inlet tube 5A is not closed by the clamp 53. Herein, first the syringe in which the blood collected by way of blood collection, etc. is stored is connected to the blood inlet port 52, and the blood is introduced to the blood storage part 2 (blood storage container 21) from this syringe via the blood inlet tube 5A. After the blood has been introduced to the blood storage part 2, the path of the blood inlet tube 5A is closed by the clamp 53.

In the first separation step S2, only the blood storage part 2 is subjected to centrifuge separation. Then, after the first centrifuge separation step S2 ends, the blood storage part-side tube 41 and component storage part-side tube 42 are connected by the connector 43, and the steps of the transfer step S3 and later are performed.

According to the apparatus 1 for separating and storing blood components according to the third embodiment, the following such effects are exerted in addition to exerting similar effects as the first embodiment and the second embodiment.

The linking tube 4A is configured to include the blood storage part-side tube 41, the component storage part-side tube 42, and the connector 43 that detachably links these. Since the blood storage part 2 and component storage part 3 can thereby be separated, only the blood storage part 2 is subjected to centrifuge separation. Consequently, since centrifuge separation of the blood storage part 2 becomes possible even when using a centrifuge that cannot accommodate a plurality of centrifuge tubes, the ease of use of the apparatus 1 for separating and storing blood components can be further improved.

In addition, the apparatus 1 for separating and storing blood components is configured to include the blood inlet port 52 provided to a base end side of the blood inlet tube 5A. Since collected blood can thereby be introduced to the blood storage part 2 by connecting a syringe in which blood is contained to the blood inlet port 52, the range of applications of the apparatus 1 for separating and storing blood components can be expanded.

The present invention has been described above based on preferred embodiments, but the present invention is not limited to the abovementioned embodiments, and changes that do not depart from the scope of the invention are possible as appropriate.

For example, the position of the leading end 371a of the metal needle 371 is explained as being fixed in the first embodiment; however, it may be configured so that the position of the leading end 371a is variable by extending or contracting the length of the metal needle 371 according to the amount of blood to be collected, etc.

In addition, the connection between the first cap 23 and the blood storage container 21 or the first storage container 22, and the connection between the second cap 33 and the component storage container 31 or the second storage container 32 in the first embodiment is by mating, but these connections may be, for example, by threading together.

Furthermore, in the first embodiment, a hermetically sealed member is interposed between the first cap 23 and the first storage container 22, but there is no limitation to this, and the hermetically sealed member may be interposed between the second cap 33 and the component storage container 31.

In addition, in the first embodiment, the lower end part of the blood storage container 21 is formed from the bottom cap 210, but the lower end part of the blood storage container 21 may be formed by welding and sealing a flexible member forming a side face, at the lower end part.

Furthermore, the bottom cap 210 may be formed by a flexible member forming a side face.

In this way, by forming the bottom cap 210 by a member that has flexibility, the blood storage container 21 deforms easily in the bottom end part due to pressurization of the pressure regulation space 26, and it is possible to easily extract blood components into the component storage part 3.

Furthermore, transport of blood component from the blood storage container 21 to the component storage container 31 in the first embodiment is performed by deforming the blood storage container 21 by pressurizing the pressure regulation space, but the blood storage container 21 may be deformed by applying a negative pressure inside the component storage container 31 and the blood storage container 21 that communicates therewith, by drawing air from the ventilation channel 38.

In addition, in the first embodiment, the component storage container 31 has a lateral face configured from a flexible material; however, it may be configured from a hard member.

In addition, the injection hole 29 in the first embodiment is provided in the first cap 23, but may be provided on a side face of the first storage container 22.

Furthermore, the lower end parts of the blood storage container 21 and the component storage container 31 in the first embodiment are configured to be at a distance from the respective inner sides of the first storage container 22 and the second storage container 32, but a configuration is also possible in which the two members are fixed by coming into contact or by means for mating together.

In addition, a configuration is possible in which a spacer (not illustrated) is disposed between the lower end part of the blood storage container 21 and the bottom part of the first storage container 22, and between the lower end part of the component storage container 31 and bottom part of the second storage container 32.

With the abovementioned configuration, in the centrifuge separation step in a blood component separation operation of the present apparatus, it is possible to reduce load placed on each of the blood storage container 21 and the component storage container 31.

Furthermore, a blood cell removing filter (not illustrated) may be interposed in an empty portion of any of the component outlet channel 28, the linking tube 4 or the component inlet channel 37. In this way, blood cell components in the blood storage container 21 can be captured by the blood cell removing filter, and it is possible to prevent mixing of the blood cell components into the component storage container 31.

In addition, in the third embodiment, the blood storage part 2 and component storage part 3 are separated in the blood introduction step S1 and first separation step S2, and in subsequent steps, the blood storage part 2 and component storage part 3 are linked; however, it is not limited thereto. In other words, after linking one end of the blood storage part 2 and component storage part 3 in the transfer step S3, for example, the blood storage part 2 and component storage part 3 are separated again in the second separation step S4, and subsequently, the blood storage part 2 and component storage part 3 may be further separated in the concentration step S5. Only the blood storage part 2 is thereby subjected to centrifuge separation, not only in the first separation step S2, but also in the second separation step S4. In this case, the path of the component storage part-side tube 42 can be closed as necessary, by providing a clamp also to the component storage part-side tube 42.

In addition, in the third embodiment, blood is introduced by connecting a syringe to the blood inlet port 52; however, it is not limited thereto. In other words, a blood bag in which blood is contained may be connected to the blood inlet port 52 via a needle or the like capable of connecting to the blood inlet port 52.

### EXPLANATION OF REFERENCE NUMERALS

- 1: apparatus for separating and storing blood components
- 2: blood storage part
- 21: blood storage container
- 22: first storage container
- 23: first cap
- 24: first connection part
- 25: second connection part
- 26: pressure regulation space
- 27: blood inlet channel
- 28: component outlet channel
- 29: injection hole
- 240: decreasing-diameter part
- 240a: convex curved face
- 3: component storage part
- 31: component storage container
- 32: second storage container
- 33: second cap
- 34: component collection orifice
- 35: third connection part
- 36: fourth connection part
- 37: component inlet channel
- 371: metal needle (return pipe)
- 38: ventilation channel
- 4, 4A: linking tube
- 41: blood storage part-side tube
- 42: component storage part-side tube
- 43: connector
- 5, 5A: blood drawing tube (blood inlet tube)
- 51: co-injection port (anticoagulant introduction part)
- 52: blood inlet port
- 6: clamp (closing-off means)
- 7: ventilation tube

## Claims

1. An apparatus for separating and storing blood components, the apparatus comprising: a blood storage part for storing blood; a component storage part for storing a part of the components of the blood stored in the blood storage part; and a linking tube for aseptically linking the blood storage part and the component storage part,
wherein the blood storage part has a blood storage container in the form of a flexible cylinder, a blood inlet channel for introducing the blood into the blood storage container, and a component outlet channel for extracting a part of the components of the blood from the blood storage container,
wherein the component storage part has a component inlet channel for introducing a part of the components of the blood that have been extracted from the blood storage container, and a return pipe that extends from the component inlet channel towards an interior space of the component storage part, capable of sending back a part of the components of the blood stored in the component storage part to the blood storage container, and
wherein the linking tube links the component outlet channel and the component inlet channel.

2. The apparatus for separating and storing blood components according to claim 1, further comprising a closing-off device that is provided to the linking tube and is for blocking movement of the part of the components of the blood within the linking tube.

3. The apparatus for separating and storing blood components according to claim 1 or 2, wherein the linking tube includes:
a blood storage part-side tube having one end side connected to the blood storage part;
a component storage part-side tube having one end side connected to the component storage part; and
a connector that detachably links the blood storage part-side tube and the component storage part-side tube.

4. The apparatus for separating and storing blood components according to any one of claims 1 to 3, further comprising:
a blood inlet tube that is connected to the blood inlet channel; and
an anticoagulant introduction part that is provided to the blood inlet tube, and leads in an anticoagulant to the blood storage container.

5. The apparatus for separating and storing blood components according to any one of claims 1 to 3, further comprising:
a blood inlet tube having a leading end side that is connected to the blood inlet channel; and
a blood inlet port that is provided to a base end side of the blood inlet tube.

6. The apparatus for separating and storing blood components according to any one of claims 1 to 5, wherein the return pipe is configured from a hard member.

7. The apparatus for separating and storing blood components according to any one of claims 1 to 6,
wherein an opening is formed in one end side of the blood storage container,
wherein the blood storage part further includes a first cap that fits the opening of the blood storage container and hermetically seals the blood storage container, and
wherein the blood inlet channel and the component outlet channel are provided to the first cap.

8. The apparatus for separating and storing blood components according to claim 7,
wherein the blood storage part further includes a first storage container having a cylindrical shape and an opening formed at one end, and containing the blood storage container,
wherein the opening of the first storage container is hermetically sealed by being fitted to the first cap, and
wherein a pressure regulation space is formed in a space independent of an internal space of the blood storage container, between an outer side of the blood storage container and an inner side of the first storage container.

9. The apparatus for separating and storing blood components according to claim 8, wherein the blood storage part comprises an injection hole by which fluid can be injected into the pressure regulation space.

10. The apparatus for separating and storing blood components according to claim 9 further comprising an injection device, linked to the injection hole, for injecting fluid into the pressure regulation space, wherein the blood storage container deforms due to the fluid that is injected.

11. The apparatus for separating and storing blood components according to claim 9 or 10, wherein the injection hole is provided in the first cap.

12. The apparatus for separating and storing blood components according to any one of claims 1 to 11, wherein the component storage part comprises: a component storage container of cylindrical shape having an opening at one end, a second storage container of cylindrical shape having an opening at one end, and containing the component storage container, and a second cap connected to the opening in the component storage container and fitting the opening in the second storage container.

13. The apparatus for separating and storing blood components according to claim 12, wherein the component storage part has a ventilation channel in which circulation of air is performed with regard to the component storage container, wherein a ventilation tube provided with a ventilation filter is further linked to the ventilation channel.

14. The apparatus for separating and storing blood components according to claim 12 or 13, wherein the component storage container comprises a component collection orifice in a bottom part that is an end part different from one end at which the opening is provided in the component storage container.

15. The apparatus for separating and storing blood components according to any one of claims 1 to 14, wherein the blood storage part and the component storage part have a self-sustaining shape.

16. The apparatus for separating and storing blood components according to any one of claims 7 to 15,
wherein the first cap further includes a decreasing-diameter part which is configured so that a lower face of the first cap is formed to be indented to a top side and gradually reduces in diameter from a lower end side of the first cap towards an upper end,
wherein the component outlet channel is connected to an upper end part of the decreasing-diameter part, and
wherein a face of the decreasing-diameter part extending from a connecting portion between the component outlet channel and the decreasing-diameter part toward a bottom end side of the first cap is configured by a convex curved face that is convex downwards.

17. A port according to claim 16, wherein a cross-section in a height direction of the decreasing-diameter part is configured by an arc-shaped curve.

18. A port for transferring blood components according to claim 16 or 17, wherein a cross-section in a direction orthogonal to the height direction of the decreasing-diameter part has a circular shape.

19. The port for transferring blood components according to any one of claims 16 to 18, wherein an angle of an upper end part of the decreasing-diameter part is 10° to 60°.

20. A method for preparing platelet-rich plasma performed using the apparatus for separating and storing blood components according to any one of claims 1 to 19, the method comprising:
a blood introduction step of introducing blood into the blood storage container through the blood inlet channel;
a first separation step of separating the blood introduced into the blood storage container in the blood introduction step into a component containing plasma and platelets, and another component;
a transfer step of transferring aseptically and hermetically the component containing plasma and platelets that was separated in the first separation step to the component storage part via the linking tube, by causing the blood storage container to deform as if being crushed by pressurizing;
a second separation step of separating the component containing plasma and platelets that was transferred to the component storage part in the transfer step into an upper layer containing plasma and a lower layer containing platelets; and
a concentration step of concentrating platelets contained in the lower layer by sending back the upper layer that was separated in the second separation step to the blood storage container via the linking tube by returning back to original a shape of the blood storage container.
